# FASCICULE DE BREVET EUROPEEN

(11) **EP 1 603 564 B1**
(45) Date de publication et mention de la délivrance du brevet: **04.07.2007**
(21) Numéro de dépôt: 04720637.0
(22) Date de dépôt: 15.03.2004
(51) Int. Cl.: A61K 31/445, A61P 25/00

(54) **UTILISATION DE DERIVES DE PYRIDIN-2-YL-METHYLAMINE POUR LE TRAITEMENT DES SYMPTOMES DE LA DOULEUR CHRONIQUE D'ORIGINE NEUROPATHIQUE OU PSYCHOGENE**
VERWENDUNG VON PYRIDIN-2-YL-METHYLAMINDERIVATEN ZUR BEHANDLUNG VON CHRONISCHEN SCHMERZ-SYMPTOMEN NEUROPATHISCHEN ODER PSYCHOGENEN URSPRUNGS.
USE OF PYRIDIN-2-YLMETHYLAMINE DERIVATIVES FOR THE PRODUCTION OF A MEDICAMENT FOR THE TREATMENT OF CHRONIC NEUROPATHOLOGICAL OR PSYCHOGENIC PAIN SYMPTOMS

(30) Priorité: 13.03.2003 FR 0303099
(43) Date de publication de la demande: 14.12.2005
(73) Titulaire: PIERRE FABRE MEDICAMENT, 92100 Boulogne (FR)
(72) Inventeur: COLPAERT, Francis, F-81700 Puylaurens (FR); VACHER, Bernard, F-81100 Castres (FR)
(74) Mandataire: Ahner, Francis
(86) Numéro de dépôt international: PCT/FR2004/000630
(87) Numéro de publication internationale: WO 2004/083171

(56) Documents cités:
- WO-A-00/21953
- WO-A-98/22459
- WO-A-03/106449
- COLPAERT, F. C. ET AL: "Large-amplitude 5-HT1A receptor activation: a new mechanism of profound, central analgesia" NEUROPHARMACOLOGY, vol. 43, 2002, pages 945-958, XP002261892
- DESEURE, KRISTOF ET AL: "The 5-HT1A receptor agonist F 13640 attenuates mechanical allodynia in a rat model of trigeminal neuropathic pain" EUROPEAN JOURNAL OF PHARMACOLOGY, no. 456, 2002, pages 51-57, XP002261893
- BRUINS SLOT, LIESBETH A. ET AL: "Tolerance and inverse tolerance to the hyperalgesic and analgesic actions, respectively, of the novel analgesic, F 13640" EUROPEAN JOURNAL OF PHARMACOLOGY, vol. 466, 2003, pages 271-279, XP002261894

## Description

La présente invention est relative à l'utilisation de dérivés de pyridin-2-yl-méthylamine pour la préparation d'un médicament destinés au traitement curatif des symptômes de la douleur chronique d'origine neuropathique ou psychogène.

Dans la demande de brevet internationale WO 98/22459, la Demanderesse a décrit les composés répondant à la formule générale (I) : 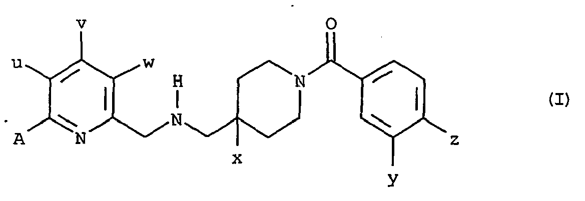 Les composés (I) sont caractérisés par leurs affinité et sélectivité au niveau des récepteurs du sous-type 5-HT1_{A} ainsi que par leur capacité à provoquer un élément particulier du syndrome sérotoninergique (i.e., la rétraction de la lèvre inférieure) chez le rat après administration orale. A ce titre, les composés (I) sont revendiqués comme médicaments potentiellement utiles pour le traitement de la dépression et de la douleur.

La présente invention a pour objet une amélioration des techniques et traitements analgésiques existants, réalisée au moyen des composés de formule (I). L'amélioration revendiquée ouvre des perspectives thérapeutiques nouvelles pour le traitement préventif et/ou curatif des symptômes douleureux chroniques pour lesquels il n'y a pas, actuellement, de solution thérapeutique satisfaisante.

On distingue généralement trois types de douleurs chroniques classées selon les mécanismes mis en cause, à savoir, les douleurs par excès de stimulations nociceptives, les douleurs neuropathiques (ou neurogènes ou de désafférentation) et les douleurs psychogènes. Certaines douleurs ont, toutefois des caractéristiques communes à plusieurs types de douleurs.

L'expression douleur chronique utilisée dans le cadre de la présente demande de brevet, regroupe les syndromes douloureux dont l'évolution dure plus de trois mois, mais dont l'intensité peut varier au cours du temps.

Dans le cadre de la présente invention, la douleur est définie comme une expérience sensorielle et émotionnelle anormale, désagréable, voire pénible, qui est perçue et intégrée au niveau le plus élevé du cortex cérébral, ce qui lui confère sa tonalité émotionnelle et affective.

Dans le cadre de la présente invention, l'expression douleur neuropathique vise l'ensemble des douleurs qui ont pour origine une ou plusieurs lésion(s) et/ou dysfonction(s) du système nerveux central ou périphérique.

Dans le cadre de la présente invention, l'expression douleur psychogène désigne généralement des douleurs qui n'ont pas de cause pathologique apparente. Il s'agit de douleurs sans véritable support anatomique, c'est-à-dire sans lésion objective, elles sont donc essentiellement psychologiques ou psychiques. Il peut ainsi s'agir par exemple, de douleurs dites "fantômes", c'est-à-dire une douleur initiale organique qui persiste après disparition de la lésion causale (mémoire de la douleur) ou d'une véritable hallucination de la douleur (douleur ressentie dans une partie du corps qui a été amputée) ou encore d'une douleur organique pour laquelle on ne retrouve aucune lésion. Ce type de douleurs peut être perçu dans des endroits du corps différents et avec des manifestations et intensités très diverses.

La présente invention vise donc le traitement des symptômes de douleurs erratiques, c'est-à-dire qui changent fréquemment de place.

La présente invention vise également le traitement d'autres douleurs telles que la douleur gravative, c'est-à-dire accompagnée d'une sensation de pesanteur, la douleur pongitive, c'est-à-dire comparable à celle qui serait provoquée par un coup de poignard, la douleur sourde, c'est-à-dire légère mais continue, la douleur tensive, c'est-à-dire accompagnée d'une sensation de distension, ou encore la douleur térébrante, c'est-à-dire intense, profonde que le patient perçoit comme si elle était produite par un corps pointu introduit dans une partie du malade.

Par médicaments analgésiques on entendra des médicaments qui soulagent ou suppriment la douleur sans entraîner la perte de sensations ou de la conscience.

En résumé, la présente invention vise le traitement des symptômes correspondant non pas à des douleurs par excès de stimulations nociceptives, mais des douleurs neuropathiques ou psychogènes qui n'ont plus un rôle physiologique, par exemple sous la forme d'un signal, mais sont devenues réellement pathologiques.

La présente invention concerne donc la préparation de médicaments destinés aux traitements préventifs et/ou curatifs des symptômes de la douleur chronique d'origine neuropathique ou psychogène.

Les médicaments utilisés en clinique humaine pour traiter les douleurs chroniques dérivent de trois familles pharmacologiques distinctes dont les chefs de file sont : la morphine, l'aspirine et le paracétamol. La morphine agit sur le système nerveux central et reste l'analgésique central de référence. L'aspirine agit sur le système nerveux périphérique, directement sur la cause de la douleur. Le paracétamol agit comme l'aspirine mais aurait aussi une action centrale. Les médicaments appartenant à ces différentes familles sont efficaces contre les douleurs par excès de stimulations nociceptives, mais peu ou pas sur les autres types de douleurs (c'est-à-dire les douleurs neuropathiques et les douleurs psychogènes). De ce fait, pour soulager certaines douleurs neuropathiques d'intensité faible ou modérée on a recours, en pratique, aux agents antidépresseurs, principalement à ceux appartenant à la classe des tricycliques (par exemple, amitriptyline, imipramine, etc.). D'autres médicaments adjuvants tels que des anxiolytiques, anticonvulsivants (par exemple, gabapentine, carbamazepine), bloqueurs de canaux sodiques (lidocaïne, mexiletine) ou glucocorticoïdes, sont aussi prescrits en association avec les morphiniques et/ou les analgésiques non morphiniques. Outre l'efficacité relativement limitée des médicaments actuels, leur utilisation chronique s'accompagnent, de plus, d'effets secondaires indésirables. On peut citer, à titre d'exemple, les problèmes de tolérance et dépendance liés à l'utilisation des morphiniques majeurs (par exemple, morphine, péthidine, etc.) ; de toxicité digestive (par exemple, aspirine et dérivés salicylés) ; d'hypotension, sédation et prise de poids (antidépresseurs tricycliques) etc.

A coté des médicaments utilisés en clinique, nombre de composés dotés de mécanismes d'action différents sont à des stades d'étude divers en tant qu'analgésique. Il s'agit, par exemple, d'agonistes 5-HT1_{A} (par exemple, WO 98/22459) ; d'inhibiteurs de recapture de la sérotonine (E-5296) ; d'inhibiteurs de recapture mixtes de sérotonine et d'adrénaline (venlafaxine) ; d'agonistes cholinergique (levetiracetam) ; d'antagonistes NMDA (memantine, CNS-5161) ; d'antagonistes du glutamate (topiramate); de modulateur des récepteurs nicotiniques (ABT-594) ; des antagonistes de CCK (colikade, devacade) ; des dérivés de capsaïcine (DA-5018, BL-1832), de cannabinoïdes (CT-3) ; des antagonistes de la nociceptine (JTC-801) etc. Ces composés sont, pour la plupart, revendiqués comme actifs dans le traitement symptomatique des douleurs neuropathiques.
Globalement, il ressort donc :
- que les traitements actuels des douleurs chroniques sont fondés essentiellement sur le traitement de la cause (par exemple, agents antiinflammatoires) et sont, de ce fait, peu ou pas efficaces contre les douleurs neuropathiques ou psychogènes ;
- que les approches thérapeutiques qui adressent le problème des douleurs neuropathiques ou psychogènes (par exemple, antidépresseurs, morphiniques) au mieux soulagent les symptômes mais n'ont aucune action curative : leur utilisation est, de plus, limitée par leurs effets secondaires ;
- que des approches nouvelles pourraient bénéficier d'un spectre d'action plus large que celui des agents conventionnels en matière de traitement symptomatique des douleurs chroniques, mais aucune ne revendique un effet qui s'apparente de quelque manière que ce soit à un effet curatif.

La présente application revendique une amélioration des traitements/techniques des syndromes douloureux chroniques d'origine neuropathique et psychogène pour lequel le besoin thérapeutique est important. Les avantages de l'amélioration de la technique qui fait l'objet de la demande, consiste :
- en ce que l'analgésie obtenue au moyen d'un agoniste 5-HT1_{A} de formule (I) s'étend au-delà du traitement symptomatique classique des douleurs chroniques d'origine neuropathique et psychogène ;
- en ce que l'analgésie obtenue au moyen d'un agoniste 5-HT1_{A} de formule (I) diminuent durablement, voire de façon permanente, le niveau d'intensité douloureuse perçue dans un contexte de douleurs neuropathiques ou psychogènes ;
- en ce que l'activité des composé de formule (I) peut être assimilée à une action curative des douleurs chroniques d'origine neuropathique ou psychogène ; à ce jour, une action de ce type n'a jamais été rapportée avec un autre agent doté de propriétés analgésiques dont, entre autres, les agonistes 5-HT1_{A}.

Sur le plan du traitement symptomatique des syndromes douloureux, certains des composés de formule (I) sont dotés d'une activité analgésique puissante dans des modèles animaux représentatifs de douleurs neuropathiques. En fait, il a déjà été établi que leur activité analgésique surpassent, généralement, celle produite par les analgésiques de référence. Cependant, au cours de l'étude de l'activité analgésique des composés (I) en administration sub-chronique et chronique, les inventeurs ont mis en évidence des propriétés inattendues dont la portée en terme d'application thérapeutique dans le traitement des douleurs chroniques peut être considérable.

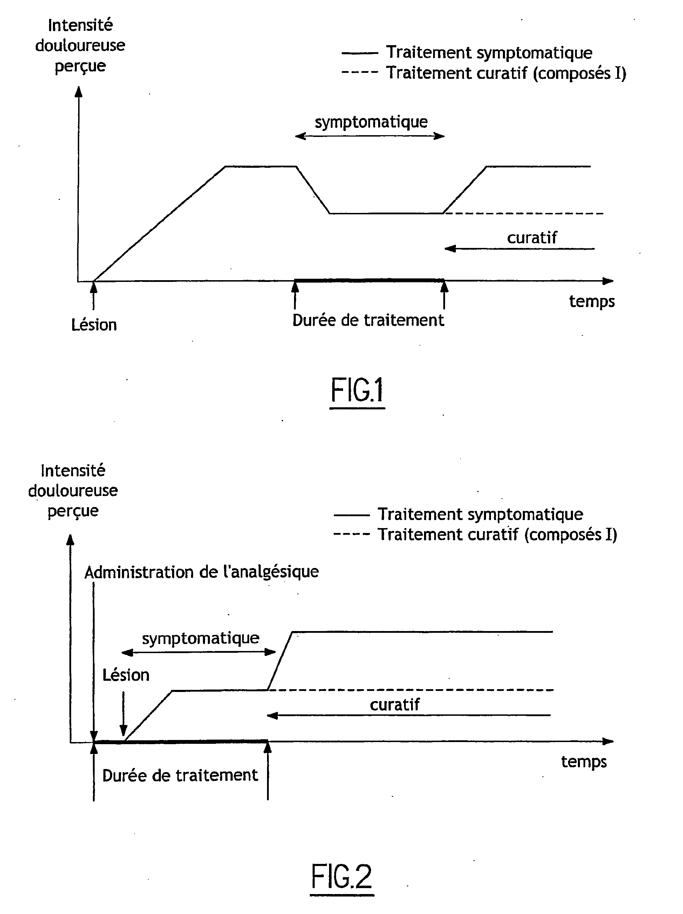

En effet, tous les agents analgésiques, quel que soit leur mode d'action, présentent un profil d'activité in vivo similaire, typique que l'on peut qualifier de symptomatique comme cela apparaît aux figures 1 et 2 annexées. Or, en plus de la phase d'analgésie symptomatique classique, les composés (I) produisent une phase d'analgésie, dite curative, qui n'a jamais été rapportée auparavant avec aucun agent ou régime analgésique. La nature de cette phase d'analgésie particulière est détaillée ci-après.

En pratique, on distingue deux schémas de traitement chronologiquement différents :
a) l'agent analgésique actif est administré pendant la phase de douleur chronique ;
b) l'administration de l'agent analgésique actif précède l'apparition de la douleur chronique.
a) l'agent analgésique actif est administré pendant la phase de douleur chronique

Ainsi, chez des animaux ayant développé une douleur chronique (hyperalgie ou allodynie) dont l'intensité a atteint son seuil d'amplitude maximale tel que cela apparaît à la figure 1 annexée, l'administration chronique d'un agent analgésique actif se traduit par une réduction significative, en principe dose dépendante, de l'intensité des symptômes douloureux perçus (c'est-à-dire, l'analgésie symptomatique). L'intensité des symptômes douloureux perçus peut être objectivée par la mesure de différents paramètres comportementaux et/ou physiologiques, conscients ou inconscients. L'effet analgésique est maintenu tant que les taux circulant de l'agent analgésique sont suffisants pour que s'exerce son activité analgésique. Lorsque le traitement est interrompu, l'intensité douloureuse perçue augmente et tend à retrouver un niveau proche de son niveau initial (c'est-à-dire, le seuil douloureux d'avant traitement). L'intensité douloureuse perçue peut aussi augmenter déjà pendant la phase de traitement si un phénomène de tolérance à l'agent analgésique se développe (par exemple, traitements avec des morphiniques majeurs).

Dans le cas de l'administration chronique de certains composés (I), on observe aussi, pendant la phase de traitement, la réduction typique, dose dépendante, de l'intensité des symptômes douloureux perçue (c'est-à-dire, l'analgésie symptomatique classique). Cependant, contrairement au cas des autres analgésiques, l'intensité de la douleur perçue ne tend pas à retrouver son niveau initial lorsque le traitement est interrompu, tel que cela apparaît à la figure 1 annexée. Ainsi, de façon tout à fait surprenante, non seulement l'analgésie persiste malgré l'arrêt du traitement mais elle se maintient à un niveau stable de même amplitude que celui atteint en fin de traitement. Le phénomène observé est d'autant plus intéressant que le composé (I) n'est plus présent dans l'organisme des animaux pendant la phase d'analgésie subséquente à l'arrêt du traitement et qu'il n'a aucun effet sur la cause de la douleur chronique (par exemple, lésion, inflammation, etc.). Dans ces conditions, l'action des composés (I) peut-être qualifiée de curative. Les mécanismes impliqués dans le mode d'action particulier des composés de formules (I), en aval de l'activation des récepteurs 5-HT1_{A} sont, à ce jour, inconnus.
b) l'administration de l'agent analgésique actif précède l'apparition de la douleur chronique

Le profil d'activité analgésique typique d'un composé actif lorsque le traitement chronique est initié avant l'induction de la douleur chez l'animal (tel que cela apparaît à la figure 2 annexée), montre que l'intensité douloureuse perçue après la lésion atteint un seuil dont l'amplitude est inférieure à celle du seuil atteint en l'absence de traitement (c'est-à-dire, effet analgésique symptomatique classique). Cette phase d'analgésie symptomatique disparaît systématiquement lorsque le traitement est interrompu (tel que cela apparaît à la figure 2 annexée).

Dans le cas de l'administration chronique de certains composés (I), lorsque le traitement est initié avant l'induction de la douleur, on observe aussi la phase d'analgésie symptomatique caractéristique des analgésiques actifs. Toutefois, comme dans le cas a) l'analgésie produite par les composés (I) persiste et se maintient à un niveau stable malgré l'interruption du traitement (tel que cela apparaît à la figure 2 annexée). Pendant cette phase d'analgésie particulière le composé (I) n'est plus présent dans l'organisme des animaux et n'a aucun effet sur la cause de la douleur chronique. Un traitement au moyen d'un composé (I) qui précède l'apparition de la douleur chronique tend donc à prévenir ou à atténuer fortement son développement ultérieur. Cet aspect particulier de l'activité des composé (I) est potentiellement avantageux pour le traitement préventif des douleurs post-opératoires. Il peut être utile, aussi, dans certaines atteintes du système nerveux dans lesquelles un délais plus ou moins important sépare la lésion de l'apparition de la douleur (par exemple, lésions rachidiennes, douleur de membre fantôme).

Le profil d'évolution unique et inattendu de l'activité analgésique des composés (I), en particulier de par leur action curative et cela quel que soit le schéma de traitement (c'est-à-dire, pré- ou post- développement de la douleur), représente une amélioration substantielle de l'état de la technique actuel.

Les composés de l'invention permettent donc, potentiellement, de répondre à un besoin thérapeutique dans le domaine du traitement des douleurs chroniques d'origine neuropathique et/ou psychogène dans lequel les médicaments disponibles s'avèrent peu ou pas efficaces ou s'avèrent d'une utilité limitée de par leur effets secondaires.

Les composés (I) sont représentés, sous forme de base, par la formule générale (I) : 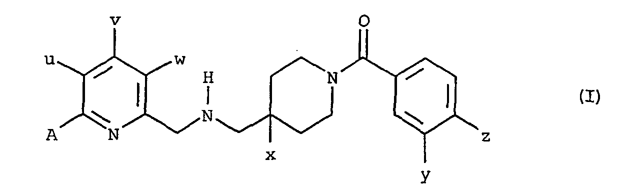 dans laquelle:
- **u**: représente un atome d'hydrogène ou un radical méthyl avec la réserve que lorsque **u** est un radical méthyl alors **v** et **w** représentent un atome d'hydrogène;
- **v**: représente un atome d'hydrogène, un atome de chlore ou un radical méthyl avec la réserve que lorsque **v** est un radical méthyl alors **u** et **w** représentent un atome d'hydrogène;
- **w**: représente un atome d'hydrogène, un atome de fluor ou un radical méthyl avec la réserve que lorsque **w** est un radical méthyl alors **u** et **v** représentent un atome d'hydrogène;
- **x**: représente un atome d'hydrogène ou un atome de fluor;
- **y**: représente un atome de chlore ou un radical méthyl;
- **z**: représente un atome d'hydrogène ou un atome de fluor ou un atome de chlore ou un radical méthyl;
- **A**: représente:
- un atome d'hydrogène ou un atome de fluor ou un atome de chlore;
- un radical alkyl en C₁-C₅, i.e. un restes d'hydrocarbures aliphatiques saturés à chaîne droite ou ramifiée, contenant 1 à 5 atomes de carbone tel que méthyl, éthyl, propyl, butyl, pentyl, isopropyl, 1-méthyl-éthyl, 1-méthyl-propyl, 1-méthyl-butyl, 2-méthyl-propyl, 2-méthyl-butyl ou 3-méthyl-butyl, 1-éthyl-propyl, 2-éthyl-propyl;
- un radical fluoroalkyl tel que monofluorométhyl (-CH₂F) ou difluorométhyl (-CHF₂) ou trifluorométhyl (-CF₃) ou 1-fluoro-1-éthyl (-CHFCH₃) ou 1,1-difluro-1-éthyl (-CF₂CH₃);
- un radical cyclopropyl ou cyclobutyl ou cyclopentyl;
- un groupe hétérocyclique aromatique à 5 chaînons, substitué ou non, contenant 1, 2, 3 ou 4 hétéroatomes choisis parmis l'azote, l'oxygène et le soufre sans toutefois que plus d'un atome d'oxygène et / ou de soufre ne soit présent dans l'hétérocycle **A.**
- Les hétérocycles aromatiques étant de préférence :
   furan-2-yl, (O.CH:CH.CH:C-) ou
   furan-3-yl, (CH:CH.O.CH:C-) ou
   1H-pyrrol-2-yl, (NH.CH:CH.CH:C-) ou
   1H-pyrrol-3-yl, (CH:CH.NH.CH:C-) ou
   1-méthyl-pyrrol-2-yl, (N(CH3).CH:CH.CH:C-) ou
   1-méthyl-pyrrol-3-yl, (CH:CH.N(CH3).CH:C-) ou
   thiophen-2-yl, (S.CH:CH.CH:C-) ou
   thiophen-3-yl, (CH:CH.S.CH:C-) ou
   pyrazol-1-yl, (N:CH.CH:CH.N-) ou
   1H-pyrazol-3-yl, (CH:CH.NH.N:C-) ou
   1H-pyrazol-4-yl, (CH:N.NH.CH:C-) ou
   1-méthyl-pyrazol-3-yl, (CH:CH.N(CH3).N:C-) ou
   imidazol-1-yl (CH:N.CH:CH.N-) ou
   1H-imidazol-2-yl, (NH.CH:CH.N:C-) ou
   1H-imidazol-4-yl, (N:CH.NH.CH:C-) ou
   oxazol-2-yl, (O.CH:CH.N:C-) ou
   oxazol-4-yl, (N:CH.O.CH:C-) ou
   oxazol-5-yl, (O.CH:N.CH:C-) ou
   isoxazol-5-yl (O.N:CH.CH:C-) ou
   isoxazol-4-yl, (CH:N.O.CH:C-) ou
   isoxazol-3-yl, (CH:CH.O.N:C-) ou
   thiazol-2-yl, (S.CH:CH.N:C-) ou
   thiazol-4-yl, (N:CH.S.CH:C-) ou
   thiazol-5-yl, (S.CH:N.CH:C-) ou
   isothiazol-5-yl, (S.N:CH.CH:C-) ou
   isothiazol-4-yl, (CH:N.S.CH:C-) ou
   isothiazol-3-yl, (CH:CH.S.N:C-) ou
   [1,2,4]triazol-1-yl, (CH:N.CH:N.N-) ou
   1H-[1,2,4]triazol-3-yl, (N:CH.NH.N:C-) ou
   [1,2,4]oxadiazol-3-yl, (N:CH.O.N:C-) ou
   [1,2,4]oxadiazol-5-yl, (O.N:CH.N:C-) ou
   5-méthyl-[1,2,4]oxadiazol-3-yl, (N:C(CH3).O.N:C-) ou 1H-tétrazol-5-yl, (NH.N:N.N:C-);
- un groupe alkoxy (R₁O-) ou alkylthio (R₁S-) dans lequel le radical R₁ représente:
   · un radical alkyl en C₁-C₅ tel que défini précédemment,
   un radical monofluorométhyl ou trifluorométhyl,
   un radical cyclopropyl ou cyclobutyl ou cyclopentyl;
- un groupe amino de type II 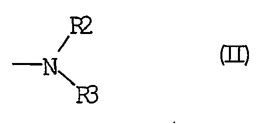 dans lequel R₂ et R₃, identiques ou différents représentent l'hydrogène, ou un radical alkyl en C₁-C₅ tel que défini précédemment ou un groupe cyclopropyl ou un groupe trifluorométhyl;
   - un groupe amino cyclique saturé de type III 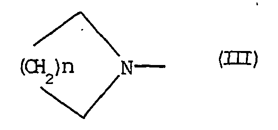 dans lequel **n** peut prendre les valeurs entières 1 ou 2;
   - un groupe alkoxycarbonyl de préférence un groupe méthoxycarbonyl (CH₃OCO-) ou un groupe éthoxycarbonyl (CH₃CH₂OCO-).

L'invention s'étend également aux sels sels d'addition des composés de formule I précitée, avec des acides minéraux ou organiques pharmaceutiquement acceptables.

La présente invention vise plus particulièrement l'utilisation des composés de Formule I choisis parmi :
(3,4-Dichloro-phényl)-(4-{[(6-pyrazol-1-yl-pyridin-2-ylméthyl)-amino]-methyl{-pipéridin-1-yl)-méthanone
(3-Chloro-4-fluoro-phényl)-(4-{[(6-pyrazol-1-yl-pyridin-2-ylméthyl)-amino]-méthyl{-pipéridin-1-yl)-méthanone
(4-Chloro-3-méthyl-phényl)-(4-{[(6-pyrazol-1-yl-pyridin-2-ylméthyl)-amino]-méthyl{-pipéridin-1-yl)-méthanone
(3-Chloro-phényl)-(4-{[(6-pyrazol-1-yl-pyridin-2-ylméthyl)-amino]-méthyl{-pipéridin-1-yl)-méthanone
(4-{[(6-Pyrazol-1-yl-pyridin-2-ylméthyl)-amino]-méthyl{pipéridin-1-yl)-m-tolyl-méthanone
(3,4-Dichloro-phényl)-(4-fluoro-4-{[(6-pyrazol-1-yl-pyridin-2-ylméthyl)-amino]-méthyl{-pipéridin-1-yl)-méthanone
(3,4-Dichloro-phényl)-(4-{[(6-imidazol-1-yl-pyridin-2-ylméthyl)-amino]-méthyl{-pipéridin-1-yl)-méthanone
(3,4-Dichloro-phényl)-(4-{{(6-[1,2,4,]triazol-1-yl-pyridin-2-ylméthyl)-amino)-méthyl{-pipéridin-1-yl)-méthanone
(3,4-Dichloro-phényl)-(4-{[(6-pyrrol-1-yl-pyridin-2-ylméthyl)-amino]-méthyl{-pipéridin-1-yl)-méthanone
(3,4-Dichloro-phényl)-(4-{[(6-méthylamino-pyridin-2-ylméthyl)-amino]-méthyl{-pipéridin-1-yl)-méthanone
(3,4-Dichloro-phényl)-(4-fluoro-4-{[(6-méthylamino-pyridin-2-ylméthyl)-amino]-méthyl{-pipéridin-1-yl)-méthanone
(3,4-Dichloro-phényl)-(4-{[(6-diméthylamino-pyridin-2-ylméthyl)-amino]-méthyl{-pipéridin-1-yl)-méthanone
(3-Chloro-4-fluoro-phényl)-(4-{[(6-diméthylamino-pyridin-2-ylméthyl)-amino]-méthyl{-4-fluoro-pipéridin-1-yl)-méthanone
(3,4-Dichloro-phényl)-(4-{[(6-diméthylamino-pyridin-2-ylméthyl)-amino]-méthyl{-4-fluoro-pipéridin-1-yl)-méthanone
(3,4-Dichloro-phényl)-[4-({[6-(éthyl-méthyl-amino)-pyridin-2-ylméthyl]-amino{-méthyl)-4-fluoro-pipéridin-1-yl]-méthanone
(3,4-Dichloro-phényl)-[4-({[6-(méthyl-propyl-amino)-pyridin-2-ylméthyl]-amino{-méthyl)-pipéridin-1-yl]-méthanone
(4-{[(6-Azétidin-1-yl-pyridin-2-ylméthyl)-amino]-méthyl{pipéridin-1-yl)-(3,4-dichloro-phényl)-méthanone
(4-{[(6-Azétidin-1-yl-pyridin-2-ylméthyl)-amino]-méthyl{-4-fluoro-pipéridin-1-yl)-(3,4-dichloro-phényl)-méthanone
(4-{[(6-Cyclopentyl-pyridin-2-ylméthyl)-amino]-méthyl{pipéridin-1-yl)-(3,4-dichloro-phényl)-méthanone
(4-{[(6-Chloro-pyridin-2-ylméthyl)-amino]-méthyl{-pipéridin-1-yl)-(3,4-dichloro-phényl)-méthanone
(3,4-Dichloro-phényl)-[4-({[6-(1H-pyrazol-3-yl-)-pyridin-2-ylméthyl]-amino{-méthyl)-pipéridin-1-yl]-méthanone
(3,4-Dichloro-phényl)-[4-fluoro-4-({[6-(1H-pyrazol-3-yl-)-pyridin-2-ylméthyl]-amino{-méthyl)-pipéridin-1-yl]-méthanone
(3,4-Dichloro-phényl)-[4-fluoro-4-({[6-(1-méthyl-pyrazol-3-yl)-pyridin-2-ylméthyl]-amino{-méthyl)-pipéridin-1-yl]-méthanone
(3,4-Dichloro-phényl)-[4-({[6-(1H-imidazol-2-yl)-pyridin-2-ylméthyl]-amino{-méthyl)-pipéridin-1-yl]-méthanone
(3,4-Dichloro-phényl)-(4-{[(6-thiazol-2-yl-pyridin-2-ylméthyl)-amino]-méthyl{-pipéridin-1-yl)-méthanone
(3,4-Dichloro-phényl)-(4-fluoro-4-{[(6-thiazol-2-yl-pyridin-2-ylméthyl)-amino]-méthyl{-pipéridin-1-yl)-méthanone
(3,4-Dichloro-phényl)-[4-({[6-(1H-pyrrol-2-yl)-pyridin-2-ylméthyl]-amino(-méthyl)-pipéridin-1-yl]-méthanone
(3,4-Dichloro-phényl)-(4-{[(6-thiophen-2-yl-pyridin-2-ylméthyl)-amino]-méthyl(-pipéridin-1-yl)-méthanone
(3,4-Dichloro-phényl)-(4-fluoro-4-{[(6-thiophen-2-yl-pyridin-2-ylméthyl)-amino]-méthyl{-pipéridin-1-yl)-méthanone
(3,4-Dichloro-phényl)-(4-([(6-furan-2 -yl-pyridin-2-ylméthyl)-amino]-méthyl{-pipéridin-1-yl)-méthanone
(3,4-Dichloro-phényl)-(4-fluoro-4-{[(6-furan-2-yl-pyridin-2-ylméthyl)-amino]-méthy{-pipéridin-1-yl)-méthanone
(3-Chloro-4-fluoro-phényl)-(4-fluoro-4-{[(6-furan-2-yl-pyridin-2-ylméthyl)-amino]-méthyl{-pipéridin-1-yl)-méthanone
(3,4-Dichloro-phényl)-(4-{[(6-oxazol-5-yl-pyridin-2-ylméthyl)-amino]-méthyl{-pipéridin-1-yl)-méthanone
(3,4-Dichloro-phényl)-(4-fluoro-4-{[(6-oxazol-5-yl-pyridin-2-ylméthyl)-amino]-méthyl{-pipéridin-1-yl)-méthanone
(3,4-Dichloro-phényl)-(4-fluoro-4-{[(6-furan-3-yl-pyridin-2-ylméthyl)-amino]-méthyl{-pipéridin-1-yl)-méthanone
(3,4-Dichloro-phényl)-[4-({[6-(5-méthyl-[1,2,4]oxadiazol-3-yl)-pyridin-2-ylméthyl]-amino{-méthyl)-pipéridin-1-yl]-méthanone
(3,4-Dichloro-phényl)-(4-{[(6-méthyl-pyridin-2-ylméthyl)-amino]-méthylf-pipéridin-1-yl)-méthanane
(3,4-Dichloro-phényl)-(4-{[(6-isopropyl-pyridin-2-ylméthyl)-amino]-méthyl{-pipéridin-1-yl)-méthanone
(3,4-Dichloro-phényl)-(4-{[(6-cyclopropyl-pyridin-2-ylméthyl)-amino]-méthyl}-4-fluoro-pipéridin-1-yl)-méthanone
(3,4-Dichloro-phényl)-(4-fluoro-4-{[(6-fluorométhyl-pyridin-2-ylméthyl)-amino]-méthyl{-pipéridin-1-yl)-méthanone
(3,4-Dichloro-phényl)-(4-{[(6-difluorométhyl-pyridin-2-ylméthyl)-amino]-méthyl{-pipéridin-1-yl)-méthanone
(3,4-Dichloro-phényl)-(4-{[(6-difluorométhyl-pyridin-2-ylméthyl)-amino]-méthyl{-4-fluoro-pipéridin-1-yl)-méthanone
(3,4-Dichloro-phényl)-[4-fluoro-4-({[6-(1-fluoro-éthyl)-pyridin-2-ylméthyl]-amino{-méthyl)-pipéridin-1-yl]-méthanone
6-({[1-(3,4-Dichloro-benzoyl)-pipéridin-4-ylméthyl]-amino{méthyl)-pyridine-2-carboxylic acid méthyl ester
6-({[1-(3,4-Dichloro-benzoyl)-pipéridin-4-ylméthyl]-amino{méthyl)-pyridine-2-carboxylic acid éthyl ester
(3,4-Dichloro-phényl)-(4-{[(6-méthoxy-pyridin-2-ylméthyl)-amino]-méthyl{-pipéridin-1-yl)-méthanone
(3,4-Dichloro-phényl)-(4-fluoro-4-{[(6-méthoxy-pyridin-2-ylméthyl)-amino]-méthyl{-pipéridin-1-yl)-méthanone
(3,4-Dichloro-phényl)-(4-fluoro-4{[(6-isopropyloxy-pyridin-2-ylméthyl)-amino)-méthyl}-pipéridin-1-yl)-méthanone
(4-{[(6-Cyclopentyloxy-pyridin-2-ylméthyl)-amino]-méthyl{pipéridin-1-yl)-(3,4-dichloro-phényl)-méthanone
(3,4-Dichloro-phényl)-(4-{[(6-méthylsulfanyl-pyridin-2-ylméthyl)-amino]-méthyl{-pipéridin-1-yl)-méthanone
(3,4-Dichloro-phényl)-(4-{[(6-fluoro-pyridin-2-ylméthyl)-amino]-méthyl{-pipéridin-1-yl)-méthanone
(3,4-Dichloro-phényl)-(4-fluoro-4-{[(6-fluoro-pyridin-2-ylméthyl)-amino]-méthyl{-pipéridin-1-yl)-méthanone
(3,4-Dichloro-phényl)-(4-fluoro-{[(3-fluoro-pyridin-2-ylméthyl)-amino]-méthyl}-pipéridin-1-yl)-méthanone
(4-{[(4-Chloro-pyridin-2-ylméthyl)-amino]-méthyl}-pipéridin-1-yl)-(3,4-dichloro-phényl)-méthanone
(3-Chloro-4-fluoro-phényl)-(4-fluoro-4-{[(5-méthyl-6-furan-2-yl-pyridin-2-ylméthyl)-amino]-méthyl}-pipëridin-1-yl)-méthanone
(3-Chloro-4-fluoro-phényl)-[4-fluoro-4-({[5-méthyl-6-(1H-pyrazol-3-yl)-2-yl-pyridin-2-ylméthyl]-amino}-méthyl)-pipéridin-1-yl]-méthanone
(3-Chloro-4-fluoro-phényl)-(4-fluoro-4-{[(5-méthyl-6-méthylamino-pyridin-2-ylméthyl)-amino]-méthyl}-pipéridin-1-yl)-méthanone
(4-{[(6-Azétidin-1-yl-pyridin-2-ylméthyl)-amino]-méthyl}-4-fluoro-pipéridin-1-yl)-(3-chloro-4-fluoro-phényl)-méthanone
(3-Chloro-4-fluoro-phényl)-(4-fluoro-4-{[(6-oxazol-5-yl-pyridin-2-ylméthyl)-amino]-méthyl}-pipéridin-1-yl)-méthanone
(3-Chloro-4-fluoro-phényl)-(4-fluoro-4-{[(6-éthylamino-pyridin-2-ylméthyl)-amino]-méthyl}-pipéridin-1-yl)-méthanone
(3-Chloro-4-fluoro-phényl)-(4-fluoro-4-{[(6-méthylamino-pyridin-2-ylméthyl)-amino]-méthyl}-pipéridin-1-yl)-méthanone
(3-Chloro-4-méthyl-phényl)-(4-fluoro-4-{[(6-diméthylamino-pyridin-2-ylméthyl)-amino]-méthyl}-pipéridin-1-yl)-méthanone
(3-Chloro-4-fluoro-phényl)-(4-fluoro-4{[(5-méthyl-6-diméthylamino-pyridin-2-ylméthyl)-amino]-méthyl}-pipéridin-1-yl)-méthanone
(3-Chloro-4-fluoro-phényl)-[4-fluoro-4-({[6-(1H-pyrazol-3-yl)-pyridin-2-ylméthyl]-amino}-méthyl)-pypéridin-1-yl]-méthanone
(3,4-Dichloro-phényl)-(4-fluoro-4-{[(3-méthyl-6-diméthylamino-pyridin-2-ylméthyl)-amino]-méthyl}-pipéridin-1-yl)-méthanone
(3-Chloro-4-fluoro-phényl)-(4-fluoro-4-{[(6-pyrazol-1-yl-pyridin-2-ylméthyl)-amino]-méthyl}-pipéridin-1-yl)-méthanone
(3,4-Dichloro-phényl)-(4-fluoro-4-{[(5-méthyl-pyridin-2-ylméthyl)-amino]-méthyl}-pipéridin-1-yl)-méthanone
(3-Chloro-4-fluoro-phényl)-(4-fluoro-4-{[(5-méthyl-pyridin-2-ylméthyl)-amino]-méthyl}-pipéridin-1-yl)-méthanone
(3-Chloro-4-fluoro-phényl)-(4-fluoro-4-{[(6-diéthylamino-pyridin-2-ylméthyl)-amino]-méthyl}-pipéridin-1-yl)-méthanone
(3-Chloro-4-fluoro-méthyl)-(4-fluoro-4-{[(5-méthyl-6-chloro-pyridin-2-ylméthyl)-amino]-méthyl}-pipéridin-1-yl)-méthanone
(3-Chloro-4-fluoro-phényl)-(4-fluoro-4-{[(4-méthyl-6-diméthylamino-pyridin-2-ylméthyl)-amino]-méthyl}-pipéridin-1-yl)-méthanone
(3-Chloro-4-fluoro-phényl)-(4-fluoro-4-{[(5-méthyl-6-pyrazol-1-yl-pyridin-2-ylméthyl)- amino]-méthyl}-pipéridin-1-yl)-méthanone
(3-Chloro-phényl)(4-fluoro-4-{[(6-diméthylamino-pyridin-2-ylméthyl)-amino]-méthyl}-pipéridin-1-yl)-méthanone

Dans le cadre de la présente invention les composés de formule (I) préférés sont le :
(3-Chloro-4-fluorophényl)-(4-fluoro-{[(5-méthyl-pyridin-2-ylméthyl)-amino]-méthyl}-pipéridin-1-yl)-méthanone, soit **A** = H, **u** = CH₃, **v** et **w** =H, **X** = F, **y** = Cl et **z** = F, et le
(3-Chloro-4-fluorophényl)-(4-fluoro-{[(5-méthyl-6-méthylamino-pyridin-2-ylméthyl)-amino]-méthyl}-pipéridin-1-yl)-méthanone, soit **A** = NHCH₃, **u** = CH₃, **v** et **w** =H, **X** = F, **y** = Cl et **z** = F
ainsi que leurs sels d'addition avec des acides minéraux ou organiques pharmaceutiquement acceptables. Ces composés sont respectivement dénommés par la suite composés (Ia) et (Ib).

La préparation des composés (Ia) et (Ib) ainsi que leurs caractéristiques analytiques sont rapportées dans le brevet WO 02/064585.

L'invention s'étend également aux compositions pharmaceutiques contenant à titre de principe actif au moins un des dérivés de formule générale (I) ou un de ses sels ou hydrates de ses sels en combinaison avec un ou plusieurs support inertes ou autres véhicules pharmaceutiquement acceptables.

Les compositions pharmaceutiques selon l'invention peuvent être, à titre d'exemple, des compositions administrables par voie orale, nasale, sublinguale, rectale, transcutanée ou parentérale. A titre d'exemple de compositions administrables par voie orale on peut citer les comprimés, les gélules, les granules, les poudres et les solutions ou suspensions orales. Des exemples de composition pharmaceutiques contenant les produits de formule (I) sont rapportés dans le brevet WO 98/22459.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière.

Parmi les douleurs chroniques neuropathiques et psychogènes potentiellement sensibles à l'action des composés de formule (I), on peut citer plus particulièrement, à titre d'exemples illustratifs, les douleurs des neuropathies périphériques ou centrales résultant de lésions nerveuses d'origine traumatique (par exemple, accident vasculaire cérébral ), métabolique (par exemple, diabète), infectieuse (par exemple, VIH, zona, herpes), toxique (par exemple, arsenic, plomb), invasive (douleur cancéreuse) ou congénitale ; les douleurs des membres fantômes ; les céphalées ; les douleurs articulaires non inflammatoires (par exemple, arthrose) ; les fibromyalgies ; les douleurs rachidiennes ; les douleurs post-opératoires ; d'autres causes de douleurs neuropathiques sont les scléroses multiples, les douleurs chroniques. Une douleur est considérée comme chronique, lorsque le patient se plaint de souffrir depuis une période excédant 6 mois. Parmi les douleurs chroniques, il convient de citer à titre d'exemple illustratif et non limitatif, outre les douleurs associées aux fibromyalgies, les douleurs provenant des tissus fibreux, des muscles, des tendons, des ligaments et autres sites, les douleurs abdominales, les diarrhées (notamment dans le cas du syndrome du colon irritable), et les douleurs chroniques du dos.

La présente invention vise en particulier le traitement du syndrome de fibromyalgie, qui est un syndrome chronique caractérisé par une sensation de douleur ou de brûlure avec enraidissement matinal, touchant principalement les tissus fibreux, articulaires et péri-articulaires, et par un sentiment de fatigue profonde. Il convient de rappeler ici que la fibromyalgie présente un ensemble de symptômes parmi lesquels on relève le plus fréquemment un sommeil non réparateur, des maux de têtes, des troubles digestifs, un état dépressif, des spasmes musculaires, des douleurs au visage, des engourdissements etc.

L'invention vise également le traitement de certains symptômes du syndrome de fatigue chronique, caractérisé par un état d'épuisement ou de fatigue se traduisant notamment par des spasmes et/ou des douleurs musculaires.

### Etude des propriétés pharmacologiques des composé (I)

La mesure de l'activité analgésique des produits (Ia) et (Ib) permet de mettre en évidence :
- l'efficacité des composés de formule (I) dans des modèles animaux représentatifs de douleurs neuropathiques ;
- leur profil d'activité analgésique unique ;
donc d'illustrer l'amélioration des techniques/traitements analgésiques qui fait l'objet de la présente invention.

Nous décrivons, à titre d'exemples, l'activité analgésique des produits (Ia) et (Ib) obtenue dans des tests de neuropathie périphérique et centrale. Ces exemples ne limitent en aucun cas la portée de l'invention.

### Illustration de l'aspect curatif de l'effet analgésique produit par les composés (I)

### 1^{ier} cas : traitement avec le composé (Ia) lorsque la douleur chronique est établie

Le test de neuropathie centrale mis en oeuvre est réalisé chez le rat selon une méthode analogue à celle décrite par Hao et Xu dans Pain 1996, 66, 279-286. Le produit testé est délivré à taux constant au moyen d'une pompe osmotique (Alzet, modèle 2ML2) pendant 14 jours. Les pompes sont implantées 28 jours après la lésion de la moelle épinière puis retirées à la fin du traitement. Dans ce modèle, il a été établi que 28 jours après la lésion, l'animal est dans une phase d'hypersensibilité chronique qui dure plusieurs mois. Dans cette expérience on mesure l'hypersensibilité des animaux lésés traités soit au moyen du composé (Ia), soit au moyen d'une solution saline. Le seuil de douleur est objectivé par la vocalisation persistante de l'animal en réponse à une stimulation d'intensité croissante du territoire sensible au moyen de poils de von Frey. L'analgésie est donc d'autant plus efficace que l'amplitude du seuil de vocalisation est élevée. Le fumarate du composé (Ia) est administrée à 0.63 mg/rat/jour. Les pompes implantées dans le groupe d'animaux témoins lésés, libèrent un solution à 0.9% de NaCl (saline)/rat/jour.

Les tests de sensibilité à la stimulation au moyen des poils de von Frey sont effectués 3 jours après l'implantation de la pompe osmotique et poursuivis régulièrement pendant 28 jours. Les doses se réfèrent au poids du composé non salifié.

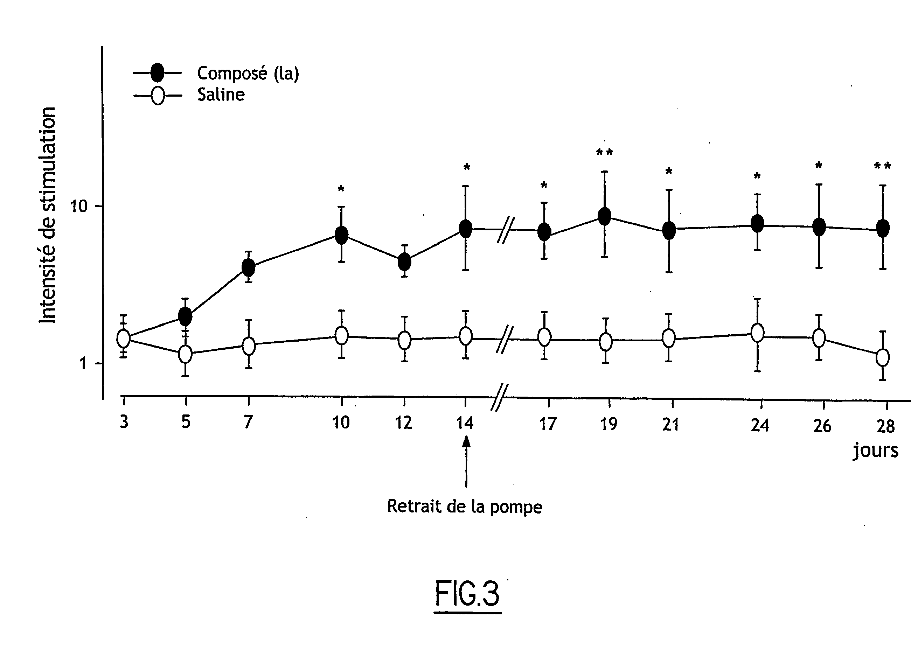

Les effets obtenues avec le composé (Ia), comparés avec ceux des animaux témoins lésés recevant une solution saline, sont représentées à la Figure 3 annexée. Dans ce modèle, les agents antidépresseurs, antiinfammatoires ainsi que les composés morphiniques sont inactifs.

### Résultats

Chez les animaux lésés, traités au moyen d'une solution saline, la stimulation qui déclenche la vocalisation est de très faible intensité et cela pendant toute la durée de l'expérience (c'est-à-dire 28 jours après lésion). Les animaux lésés ont donc développés une hypersensibilité chronique. Les animaux traités au moyen du composé (Ia) supportent une stimulation d'intensité supérieure à celle des animaux témoins et cela à tous les points de mesure pendant toute la durée du traitement. Cet état, assimilé à une analgésie, est statistiquement significatif à partir du 10^{ième} jour de traitement. Le composé Ia est donc doté d'une activité analgésique symptomatique. Après l'interruption du traitement (c'est-à-dire, à partir du jour 15 dans la figure 3 donnée en annexe), l'analgésie persiste et se maintient au même niveau que celui atteint en fin de traitement et cela significativement à tous les temps de mesure pendant le reste de l'expérience (c'est-à-dire, 14 jours après le retrait de la pompe).

Le composé Ia est donc aussi doté d'une activité analgésique curative.

Les résultats de l'expérience montrent qu'un traitement au moyen des composés I produit :
- une phase d'analgésie symptomatique ;
- une phase d'analgésie curative.

### 2^{ième} cas : traitement avec le composé (Ia) et (Ib) lorsque la douleur ne s'est pas encore développée

Le test de neuropathie périphérique mis en oeuvre est réalisé chez le rat selon une méthode analogue à celle décrite par Vos dans J. Neurosci. 1994, 14, 2708-2723. Le produit testé est délivré à taux constant (0.12 ml/jour) au moyen d'une pompe osmotique (Alzet, modèle 2ML2) pendant 14 jours. Les pompes sont implantées 24 heures avant la lésion (ligature unilatérale du nerf infraorbital) puis retirées à la fin du traitement. L'implantation des pompes est effectuée en sous-cutané à travers une incision transversale effectuée dans la peau de la face dorsale du rat. L'évaluation de l'intensité douloureuse perçue est objectivée à partir d'une série de signes comportementaux observés en réponse à une stimulation mécanique du territoire lésé au moyen de poils de von Frey. Les stimulations sont appliquées dans un ordre d'intensité croissant. L'intensité douloureuse perçue est évaluée à partir des critères suivantes : **0***, pas de réponse* de *l'animal ;* **1,** détection (i.e., le rat tourne la tête vers l'objet utilisé pour la stimulation) ; **2**, retrait (i.e., le rat détourne ou a un mouvement de retrait de la tête pour éviter l'objet utilisé pour la stimulation) ; **3**, *fuite ou attaque* (i.e., le rat évite l'objet par un mouvement du corps ou attaque délibérément l'objet utilisé pour la stimulation) ; **4**, *frottement de la face* (i.e,, le rat frotte à l'endroit de la stimulation, pour être pris en compte il doit y avoir au moins trois séries répétées de frottements). Le niveau de réponse des animaux non lésés correspond à 0 ou 1.

Le fumarate du composé (Ia) est administrée à 0.63 mg/rat/jour ; le glycolate du composé (Ib) à 0.16 mg/rat/jour ; l'hydrochlorure de morphine à 5 mg/rat/jour et le baclofène à 1.06 mg/rat/jour. Tous les composés sont administrés en solution dans l'eau distillée. Les pompes implantées chez les animaux témoins libèrent un solution à 0.9% de NaCl (saline)/rat/jour ; les doses se réfèrent au poids du composé non salifié.

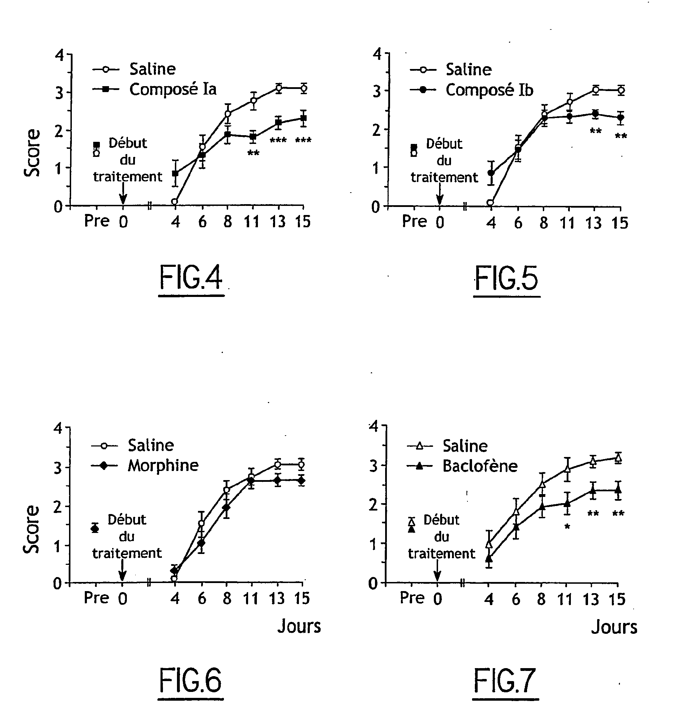

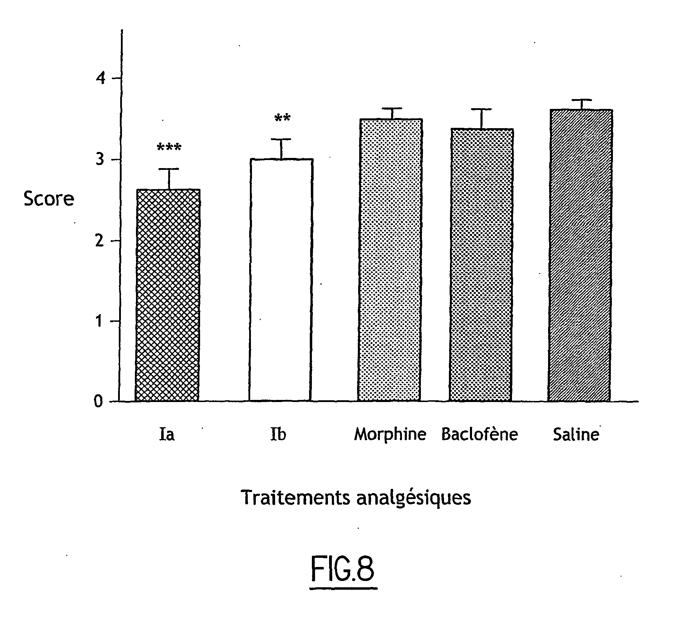

Les effets obtenus avec les composés (Ia), (Ib) et les produits choisis comme références sont représentés aux Figures 4 à 8 annexées. Les Figures 4 à 7 représentent les effets analgésiques des différents produits évalués aux jours 4, 6, 8, 11, 13 de traitement et 1 jour après retrait de la pompe (jour 15). La Figure 8 représente les effets analgésiques des différents produits mesurés au jour 21, soit 6 jours après l'arrêt du traitement. Dans ce modèle, il a été établi préalablement que les agents antidépresseurs sont inactifs alors que le baclofène est l'agent le plus actif.

Les scores individuels de chaque animal représentent la moyenne (± erreur standard) obtenue après application des poils de von-Frey. La comparaison des résultats entre les différents groupes ainsi que l'évolution dans le temps des valeurs obtenues est réalisée avec un test d'analyse de variance (ANOVA), suivi d'un test de multiple comparaison de Dunnett.

### Résultats

Les effets analgésiques observés varient au cours du temps d'une manière différente en fonction des produits évalués. La résolution du système d'évaluation adopté (critères d'évaluation de 0 à 4) a pour conséquence une faible variation relative entre les amplitudes des effets analgésiques observés.

La comparaison entre le produit (Ia) et saline montre que le composé (Ia) réduit l'hypersensibilité (allodynie) de l'animal à la stimulation (assimilée à un effet analgésique), et cela significativement à partir du jour 11 (Figure 4). Le composé (Ib) ne réduit significativement l'hypersensibilité qu'à partir du jour 13 (Figure 5). La morphine est inactive dans les conditions de l'expérience (Figure 6) alors que le baclofène a un effet analgésique significatif à partir, lui aussi, du jour 11 (Figure 7).

Six jours après arrêt du traitement, les animaux traités avec les composés (Ia) et (Ib) montrent encore un effet analgésique significatif alors que les effets de la morphine et du baclofène ont totalement disparus (Figure 8). De plus, l'amplitude de l'analgésie produite par les composés (Ia) et (Ib) mesurée 6 jours après l'interruption du traitement n'est statistiquement pas différente de celle mesurée en fin de traitement (i.e., jour 15).

Les résultats de l'expérience montrent qu'un traitement au moyen des composés (Ia) et (Ib) :
- diminue l'intensité douloureuse perçue après une lésion nerveuse d'origine périphérique et cela avec une amplitude au moins équivalente à celle du produit de référence reconnu comme le plus efficace dans ce modèle de douleur neuropathique (i.e., baclofène) ;
- l'effet analgésique produit, persiste et reste stable pendant 6 jours au moins après l'arrêt du traitement et cela contrairement à ce que l'on observe avec le baclofène.

Il ressort des études que certains composés de formule (I), ainsi que leur sel d'addition avec des acides minéraux ou les acides organiques pharmaceutiquement acceptables, sont efficaces dans le traitement des douleurs neuropathiques chroniques d'origine périphérique et centrale. L'analgésie qu'ils procurent a, de plus, un profil d'évolution unique et tout à fait remarquable. Ainsi, l'analgésie persiste et se maintient à un niveau stable très au-delà de la période de traitement et cela quel que soit le schéma de traitement (pré- ou post-lésionnel). Cette phase d'analgésie particulière, appelée curative dans la présente invention, est, contrairement à la phase symptomatique, spécifique des composés (I). Un tel profil d'évolution de l'analgésie n'a jamais été mis en évidence, non seulement avec des agonistes 5-HT_{1A}, mais aussi avec les autres agents ayant des propriétés analgésiques. A ce titre, les composés de formule (I) permettent clairement une amélioration des traitements actuels en matière d'analgésie.

## Revendications

1. Utilisation d'un composé de formule (I) 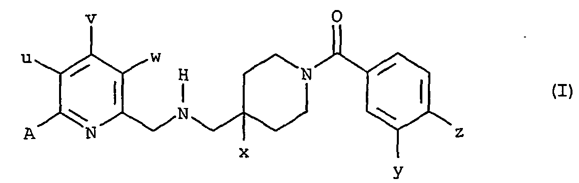 dans laquelle:
**u** représente un atome d'hydrogène ou un radical méthyl avec la réserve que lorsque **u** est un radical méthyl alors **v** et **w** représentent un atome d'hydrogène;
**v** représente un atome d'hydrogène, un atome de chlore ou un radical méthyl avec la réserve que lorsque **v** est un radical méthyl alors **u** et **w** représentent un atome d'hydrogène;
**w** représente un atome d'hydrogène, un atome de fluor ou un radical méthyl avec la réserve que lorsque **w** est un radical méthyl alors **u** et **v** représentent un atome d'hydrogène;
**x** représente un atome d'hydrogène ou un atome de fluor;
**y** représente un atome de chlore ou un radical méthyl;
**z** représente un atome d'hydrogène ou un atome de fluor ou un atome de chlore ou un radical méthyl;
**A** représente:
- un atome d'hydrogène ou un atome de fluor ou un atome de chlore;
- un radical alkyl en C₁-C₅, i.e. un restes d'hydrocarbures aliphatiques saturés à chaîne droite ou ramifiée, contenant 1 à 5 atomes de carbone tel que méthyl, éthyl, propyl, butyl, pentyl, isopropyl, 1-méthyl-éthyl, 1-méthyl-propyl, 1-méthyl-butyl, 2-méthyl-propyl, 2-méthyl-butyl ou 3-méthyl-butyl, 1-éthyl-propyl, 2-éthyl-propyl;
- un radical fluoroalkyl tel que monofluorométhyl (-CH₂F) ou difluorométhyl (-CHF₂) ou trifluorométhyl (-CF₃) ou 1-fluoro-1-éthyl (-CHFCH₃) ou 1,1-difluro-1-éthyl (-CF₂CH₃);
- un radical cyclopropyl ou cyclobutyl ou cyclopentyl;
- un groupe hétérocyclique aromatique à 5 chaînons, substitué ou non, contenant 1, 2, 3 ou 4 hétéroatomes choisis parmis l'azote, l'oxygène et le soufre sans toutefois que plus d'un atome d'oxygène et / ou de soufre ne soit présent dans l'hétérocycle **A.**
- les hétérocycles aromatiques étant de préférence :
furan-2-yl, (O.CH:CH.CH:C-) ou
furan-3-yl, (CH:CH.O.CH:C-) ou
1H-pyrrol-2-yl, (NH.CH:CH.CH:C-) ou
1H-pyrrol-3-yl, (CH:CH.NH.CH:C-) ou
1-methyl-pyrrol-2-yl, (N(CH3).CH:CH.CH:C-) ou
1-methyl-pyrrol-3-yl, (CH:CH.N(CH3).CH:C-) ou
thiophen-2-yl, (S.CH:CH.CH:C-) ou
thiophen-3-yl, (CH:CH.S.CH:C-) ou
pyrazol-1-yl, (N:CH.CH:CH.N-) ou
1H-pyrazol-3-yl, (CH:CH.NH.N:C-) ou
1H-pyrazol-4-yl, (CH:N.NH.CH:C-) ou
1-methyl-pyrazol-3-yl, (CH:CH.N(CH3).N:C-) ou
imidazol-1-yl, (CH:N.CH:CH.N-) ou
1H-imidazol-2-yl, (NH.CH:CH.N:C-) ou
1H-imidazol-4-yl, (N:CH.NH.CH:C-) ou
oxazol-2-yl, (O.CH:CH.N:C-) ou
oxazol-4-yl, (N:CH.O.CH:C-) ou
oxazol-5-yl, (O.CH:N.CH:C-) ou
isoxazol-5-yl, (O.N:CH.CH:C-) ou
isoxazol-4-yl, (CH:N.O.CH:C-) ou
isoxazol-3-yl, (CH:CH.O.N:C-) ou
thiazol-2-yl, (S.CH:CH.N:C-) ou
thiazol-4-yl, (N:CH.S.CH:C-) ou
thiazol-5-yl, (S.CH:N.CH:C-) ou
isothiazol-5-yl, (S.N:CH.CH:C-) ou
isothiazol-4-yl, (CH:N.S.CH:C-) ou
isothiazol-3-yl, (CH:CH.S.N:C-) ou
[1,2,4]triazol-1-yl, (CH:N.CH:N.N-) ou
1H-[1,2,4]triazol-3-yl, (N:CH.NH.N:C-) ou
[1,2,4]oxadiazol-3-yl, (N:CH.O.N:C-) ou
[1,2,4]oxadiazol-5-yl, (O.N:CH.N:C-) ou
5-methyl-[1,2,4]oxadiazol-3-yl, (N:C(CH3).O.N:C-) ou
1H-tetrazol-5-yl, (NH.N:N.N:C-);
- un groupe alkoxy (R₁O-) ou alkylthio (R₁S-) dans lequel le radical R₁ représente:
· un radical alkyl en C₁-C₅ tel que défini précédemment,
· un radical monofluorométhyl ou trifluorométhyl,
· un radical cyclopropyl ou cyclobutyl ou cyclopentyl;
- un groupe amino de type II 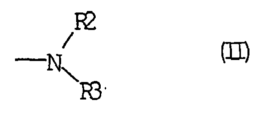 dans lequel R₂ et R₃, identiques ou différents représentent l'hydrogène, ou un radical alkyl en C₁-C₅ tel que défini précédemment ou un groupe cyclopropyl ou un groupe trifluorométhyl;
- un groupe amino cyclique saturé de type III 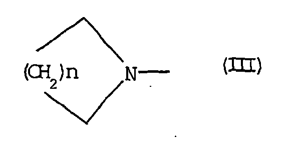 dans lequel n peut prendre les valeurs entières 1 ou 2;
- un groupe alkoxycarbonyl de préférence un groupe méthoxycarbonyl (CH₃OCO-) ou un groupe éthoxycarbonyl (CH₃CH₂OCO-) ;
ainsi que de ses sels d'addition avec des acides minéraux ou organiques pharmaceutiquement acceptables, pour la préparation d'un médicament destiné au traitement curatif des symptômes de la douleur chronique d'origine neuropathique ou psychogène.

2. Utilisation, selon la revendication 1, **caractérisée en ce que** le composé (I) répond à la formule (I) dans laquelle : **A** est choisi parmi un atome d'hydrogène ou un groupe aminométhyle, **u** représente un radical méthyle CH₃, **v** et **w** sont, tous les deux, un atome d'hydrogène, **X** représente un atome de fluor, **y** est un atome de chlore et **z** un atome de fluor.

3. Utilisation, selon l'une des revendications 1 et 2,
**caractérisée en ce que** ledit médicament est destiné au traitement préventif et curatif des symptômes douloureux chroniques d'origine neuropathique ou psychogène.

## Claims

1. Use of a compound of formula (I) 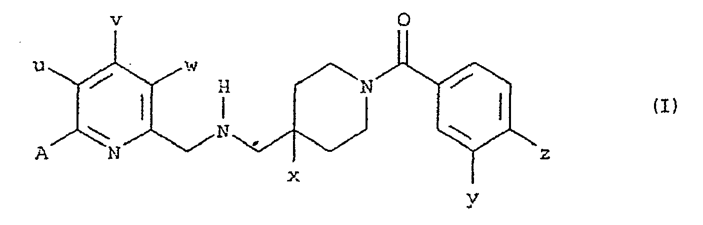 in which:
u represents a hydrogen atom or a methyl radical, with the proviso that when u is a methyl radical, then v and w represent a hydrogen atom;
v represents a hydrogen atom, a chlorine atom or a methyl radical, with the proviso that when v is a methyl radical, then u and w represent a hydrogen atom;
w represents a hydrogen atom, a fluorine atom or a methyl radical, with the proviso that when w is a methyl radical, then u and v represent a hydrogen atom;
x represents a hydrogen atom or a fluorine atom;
y represents a chlorine atom or a methyl radical;
z represents a hydrogen atom, a fluorine atom, a chlorine atom or a methyl radical;
A represents:
- a hydrogen atom, a fluorine atom or a chlorine atom;
- a C₁-C₅ alkyl radical, i.e. a straight-chain or branched-chain saturated aliphatic hydrocarbon residue containing from 1 to 5 carbon atoms, such as methyl, ethyl, propyl, butyl, pentyl, isopropyl, 1-methylethyl, 1-methylpropyl, 1-methylbutyl, 2-methylpropyl, 2-methylbutyl, 3-methylbutyl, 1-ethylpropyl or 2-ethylpropyl;
- a fluoroalkyl radical such as monofluoromethyl (-CH₂F) or difluoromethyl (-CHF₂) or trifluoromethyl (-CF₃) or 1-fluoro-1-ethyl (-CHFCH₃) or 1,1-difluoro-1-ethyl (CF₂CH₃);
- a cyclopropyl, cyclobutyl or cyclopentyl radical;
- a substituted or unsubstituted 5-membered aromatic heterocyclic group containing 1, 2, 3 or 4 hetero atoms chosen from nitrogen, oxygen and sulfur, without, however, more than one oxygen and/or sulfur atom being present in the heterocycle A;
- the aromatic heterocycles preferably being:
furan-2-yl, (O.CH:CH.CH:C-) or
furan-3-yl, (CH:CH.O.CH:C-) or
1H-pyrrol-2-yl, (NH.CH:CH.CH:C-) or
1H-pyrrol-3-yl, (CH:CH.NH.CH:C-) or
1-methylpyrrol-2-yl, (N(CH3).CH:CH.CH:C-) or
1-methylpyrrol-3-yl, (CH:CH.N(CH3).CH:C-) or
thiophen-2-yl, (S.CH:CH.CH:C-) or
thiophen-3-yl, (CH:CH.S.CH:C-) or
pyrazol-1-yl, (N:CH.CH:CH.N-) or
1H-pyrazol-3-yl, (CH:CH.NH.N:C-) or
1H-pyrazol-4-yl, (CH:N.NH.CH:C-) or
1-methylpyrazol-3-yl), (CH:CH.N(CH3).N:C-) or
imidazol-1-yl (CH:N.CH:CH.N-) or
1H-imidazol-2-yl), (NH.CH:CH.N:C-) or
1H-imidazol-4-yl), (N:CH.NH.CH:C-) or
oxazol-2-yl, (O.CH:CH.N:C-) or
oxazol-4-yl, (N:CH.O.CH:C-) or
oxazol-5-yl, (O.CH:N.CH:C-) or
isoxazol-5-yl (O.N:CH.CH:C-) or
isoxazol-4-yl (CH:N.O.CH:C-) or
isoxazol-3-yl (CH:CH.O.N:C-) or
thiazol-2-yl, (S.CH:CH.N:C-) or
thiazol-4-yl, (N:CH.S.CH:C-) or
thiazol-5-yl, (S.CH:N.CH:C-) or
isothiazol-5-yl, (S.N:CH.CH:C-) or
isothiazol-4-yl, (CH:N.S.CH:C-) or
isothiazol-3-yl, (CH:CH.S.N:C-) or
[1,2,4]triazol-1-yl, (CH:N.CH:N.N-) or
1H-[1,2,4]triazol-3-yl, (N:CH.NH.N:C-) or
[1,2,4]oxadiazol-3-yl, (N:CH.O.N:C-) or
[1,2,4]oxadiazol-5-yl, (O.N:CH.N:C-) or
5-methyl-[1,2,4]oxadiazol-3-yl), (N:C(CH3).O.N:C-) or
1H-tetrazol-5-yl, (NH.N:N.N:C-);
- an alkoxy group (R₁O-) or alkylthio group (R₁S-) in which the radical R₁ represents:
. a C₁-C₅ alkyl radical as defined above,
. a monofluoromethyl or trifluoromethyl radical,
. a cyclopropyl, cyclobutyl or cyclopentyl radical;
- an amino group of type II 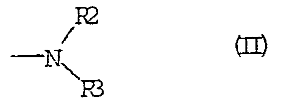 in which R₂ and R₃, which may be identical or different, represent hydrogen, a C₁-C₅ alkyl radical as defined above, a cyclopropyl group or a trifluoromethyl group;
- a saturated cyclic amino group of type III 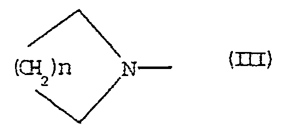 in which n may take the whole values 1 or 2;
- an alkoxycarbonyl group, preferably a methoxycarbonyl group (CH₃OCO-) or an ethoxycarbonyl group (CH₃CH₂OCO-) ;
and also of the addition salts thereof with pharmaceutically acceptable mineral or organic acids, for the preparation of a medicament for the curative treatment of the symptoms of chronic pain of neuropathic or psychogenic origin.

2. Use according to Claim 1, **characterized in that** the compound (I) corresponds to formula (I) in which: A is chosen from a hydrogen atom and an aminomethyl group, u represents a methyl radical CH₃, v and w are both a hydrogen atom, x represents a fluorine atom, y is a chlorine atom and a z is a fluorine atom.

3. Use according to either of Claims 1 and 2, **characterized in that** the said medicament is for the preventive and curative treatment of chronic pain symptoms of neuropathic or psychogenic origin.

## Patentansprüche

1. Verwendung einer Verbindung der Formel (I) 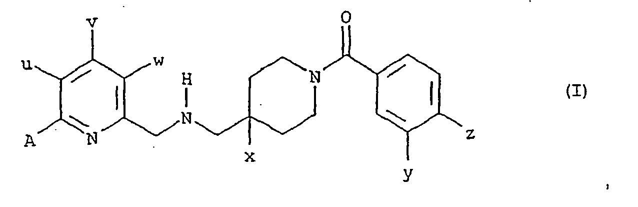 in welcher
u für ein Wasserstoffatom oder einen Methylrest steht mit der Maßgabe, dass, wenn u ein Methylrest ist, dann v und w für ein Wasserstoffatom stehen;
v für ein Wasserstoffatom, ein Chloratom oder einen Methylrest steht mit der Maßgabe, dass, wenn v ein Methylrest ist, dann u und w für ein Wasserstoffatom stehen;
w für ein Wasserstoffatom, ein Fluoratom oder einen Methylrest steht mit der Maßgabe, dass, wenn w ein Methylrest ist, dann u und v für ein Wasserstoffatom stehen;
x für ein Wasserstoffatom oder ein Fluoratom steht;
y für ein Chloratom oder einen Methylrest steht;
**z** für ein Wasserstoffatom oder ein Fluoratom oder ein Chloratom oder einen Methylrest steht,
A steht für:
- ein Wasserstoffatom oder ein Fluoratom oder ein Chloratom;
- einen C₁-C₅-Alkylrest, d.h. einen geradkettigen oder verzweigten, gesättigten aliphatischen Kohlenwasserstoffrest, welcher 1 bis 5 Kohlenstoffatome enthält, wie Methyl, Ethyl, Propyl, Butyl, Pentyl, Isopropyl, 1-Methylethyl, 1-Methylpropyl, 1-Methylbutyl, 2-Methylpropyl, 2-Methylbutyl oder 3-Methylbutyl, 1-Ethylpropyl, 2-Ethylpropyl;
- einen Fluoralkylrest, wie Monofluormethyl (-CH₂F) oder Difluormethyl (-CHF₂) oder Trifluormethyl (-CF₃) oder 1-Fluor-1-ethyl (-CHFCH₃) oder 1,1-Difluor-1-ethyl (-CF₂CH₃);
- einen Cyclopropyl- oder Cyclobutyl- oder Cyclopentylrest;
- eine substituierte oder nicht substituierte heterocyclische aromatische Gruppe mit 5 Ringatomen, welche 1, 2, 3 oder 4 Heteroatome, ausgewählt unter Stickstoff, Sauerstoff und Schwefel, enthält, wobei gleichwohl nicht mehr als ein Sauerstoffatom und/oder Schwefelatom in dem Heterocyclus A vorhanden ist.
- wobei die aromatischen Heterocyclen bevorzugt sind:
Furan-2-yl, (O.CH:CH.CH:C-) oder
Furan-3-yl, (CH:CH.O.CH:C-) oder
1H-Pyrrol-2-yl, (NH.CH:CH.CH:C-) oder
1H-Pyrrol-3-yl, (CH:CH.NH.CH:C-) oder
1-Methylpyrrol-2-yl, (N(CH₃).CH:CH.CH:C-) oder
1-Methylpyrrol-3-yl, (CH:CH.N(CH₃).CH:C-) oder
Thiophen-2-yl, (S.CH:CH.CH:C-) oder
Thiophen-3-yl, (CH:CH.S.CH:C-) oder
Pyrazol-1-yl, (N:CH.CH:CH.N-) oder
1H-Pyrazol-3-yl, (CH:CH.NH.N:C-) oder
1H-Pyrazol-4-yl, (CH:N.NH.CH:C-) oder
1-Methylpyrazol-3-yl, (CH:CH.N(CH₃).N:C-) oder
Imidazol-1-yl, (CH:N.CH:CH.N-) oder
1H-Imidazol-2-yl, (NH.CH:CH.N:C-) oder
1H-Imidazol-4-yl, (N:CH.NH.CH:C-) oder
Oxazol-2-yl, (O.CH:CH.N:C-) oder
Oxazol-4-yl, (N:CH.O.CH:C-) oder
Oxazol-5-yl, (O.CH:N.CH:C-) oder
Isoxazol-5-yl, (O.N:CH.CH:C-) oder
Isoxazol-4-yl, (CH:N.O.CH:C-) oder
Isoxazol-3-yl, (CH:CH.O.N:C-) oder
Thiazol-2-yl, (S.CH:CH.N:C-) oder
Thiazol-4-yl, (N:CH.S.CH:C-) oder
Thiazol-5-yl, (S.CH:N.CH:C-) oder
lsothiazol-5-yl, (S.N:CH.CH:C-) oder
lsothiazol-4-yl, (CH:N.S.CH:C-) oder
Isothiazol-3-yl, (CH:CH.S.N:C-) oder
[1,2,4]-Triazol-1-yl, (CH:N.CH:N.N-) oder
1H-[1,2,4]-Triazol-3-yl, (N:CH.NH.N:C-) oder
[1,2,4]-Oxadiazol-3-yl, (N:CH.O.N:C-) oder
[1,2,4]-Oxadiazol-5-yl, (O.N:CH.N:C-) oder
5-Methyl-[1,2,4]-oxadiazol-3-yl, (N:C(CH₃).O.N:C-) oder
1H-Tetrazol-5-yl, (NH.N:N.N:C-);
- eine Alkoxygruppe (R₁O-) oder Alkylthiogruppe (R₁S-), in welchen der Rest R₁ steht für:
· einen C₁-C₅-Alkylrest, wie zuvor definiert,
· einen Monofluormethyl- oder Trifluormethylrest;
· einen Cyclopropyl- oder Cyclobutyl- oder Cyclopentylrest;
- eine Aminogruppe vom Typ II 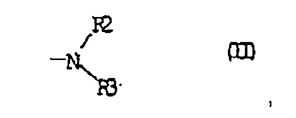 in welcher R₂ und R₃, die gleich oder verschieden sind, für Wasserstoff oder einen C₁-C₅-Alkylrest, wie zuvor definiert, oder eine Cyclopropylgruppe oder eine Trifluormethylgruppe stehen;
- eine gesättigte cyclische Aminogruppe vom Typ III 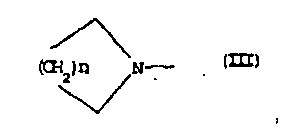 in welcher n die ganzzahligen Werte 1 oder 2 annehmen kann;
- eine Alkoxycarbonylgruppe, vorzugsweise eine Methoxycarbonylgruppe (CH₃OCO-) oder eine Ethoxycarbonylgruppe (CH₃CH₂OCO-);
wie auch von deren Additionssalzen mit pharmazeutisch annehmbaren anorganischen oder organischen Säuren für die Herstellung eines Arzneimittels, welches für die kurative Behandlung von Symptomen von chronischem Schmerz neuropathischen oder psychogenen Ursprungs bestimmt ist.

2. Verwendung nach Anspruch 1, **dadurch gekennzeichnet, dass** die Verbindung (I) der Formel (I) entspricht, in welcher: A unter einem Wasserstoffatom oder einer Aminomethylgruppe ausgewählt wird, u für einen Methylrest CH₃ steht, **v** und **w** alle beide ein Wasserstoffatom sind, x für ein Fluoratom steht, y ein Chloratom und z ein Fluoratom ist.

3. Verwendung nach einem der Ansprüche 1 und 2, **dadurch gekennzeichnet, dass** das Arzneimittel für die präventive und kurative Behandlung von chronischen Schmerzsymptomen neuropathischen oder psychogenen Ursprungs bestimmt ist.
